# EUROPEAN PATENT APPLICATION

(11) **EP 3 912 995 A1**
(43) Date of publication of application: **24.11.2021**
(21) Application number: 19910369.8
(22) Date of filing: 13.11.2019
(51) Int. Cl.: C07K 16/00, C12N 15/115, G01N 33/48, G01N 33/53, G01N 33/533, G01N 33/536, G01N 33/574

(54) **PHOSPHOR-INTEGRATED NANOPARTICLES FOR TARGET SUBSTANCE DETECTION**

(30) Priority: 18.01.2019 JP 2019006921
(71) Applicant: KONICA MINOLTA, INC., Tokyo 100-7015 (JP)
(72) Inventor: ISODA Takeshi, Tokyo 100-7015 (JP)
(74) Representative: Henkel & Partner mbB
(86) International application number: PCT/JP2019/044469
(87) International publication number: WO 2020/148985

(57) **Abstract**

[Problem] To provide phosphor-integrated nanoparticles for target substance detection having improved staining performance for labeling and detecting a target protein at high accuracy. [Solution] The phosphor-integrated nanoparticles for target substance detection are phosphor-integrated nanoparticles for target substance detection obtained by surface modifying phosphor-integrated nanoparticles by a surface modification molecule, the surface modification molecule being at least one surface modification molecule selected from the group consisting of single-chain antibodies and aptamers containing a heavy chain variable region.

## Description

### Technical Field

The present invention relates to phosphor-integrated nanoparticles for target substance detection.

### Background Art

Pathological diagnosis is a method for staining a specimen sample prepared using a tissue or a cell collected from a specimen by a predetermined method, observing the specimen sample with a microscope, examining a morphology of the tissue or the cell and an expression state of a specific biological substance such as a nucleic acid or a protein, and diagnosing the type of disease, a state of the disease, a prognosis of the disease, and various events such as an effect of a specific therapeutic agent.

In a case of pathological diagnosis of a tumor tissue, an immunohistochemistry method (IHC method) for labeling a target protein specifically expressed in the tumor tissue is widely adopted. In the IHC method, a color developing method is generally used in which an enzyme-labeled antibody is directly or indirectly bonded to a target protein using an antigen-antibody reaction, and then a substrate develops a color due to an enzyme substrate reaction that causes the enzyme to react with a substrate corresponding to the enzyme. In the color developing method, examples of a typical enzyme include horseradish peroxidase (HRP), and examples of a typical substrate include 3,3'-diaminobenzidine (DAB). Also in a case other than pathological diagnosis, an analysis kit or the like using a FACS method using an immune reaction to blood or the like collected from a specimen, an ELISA method, or the like has been commercialized. Diagnosis of various events using these is known.

However, in such a scene of diagnosis or evaluation, when the color developing method using an enzyme substrate reaction with HRP and DAB is used, a color development concentration is affected by conditions such as temperature and time. Therefore, it is difficult to accurately grasp the amount of a target protein from the color development concentration.

Under such circumstances, in pathological diagnosis, a labeling method using phosper integrated dots nanoparticles (hereinafter referred to as "PID", also referred to as fluorescent substance-integrated nanoparticles in English), which are nanoparticles each obtained by integrating a plurality of particles of a phosphor such as an organic fluorescent dye or semiconductor nanoparticles (quantum dots), has been proposed. According to this method, when a tissue, a cell, or the like stained with PID is irradiated with predetermined excitation light, the PID contained in the tissue or the like emits fluorescence. Therefore, a labeled target protein can be observed as a bright spot with high brightness. In addition, PID is less likely to cause fading than an existing dye, and therefore can be observed and imaged for a longer period of time than before.

As the PID, for example, Patent Literature 1 discloses phosphor-integrated nanoparticles each having a predetermined particle size and containing a biomolecule such as streptavidin on a surface of the particles at a predetermined ratio. Patent Literature 2 discloses phosphor-integrated nanoparticles in which streptavidin having an SH group obtained by reducing a disulfide bond of an antibody and an SH group obtained by reducing a disulfide bond with 2-iminothiolane is bonded to a surface of the particles at a predetermined ratio.

Patent Literature 1: WO 2015/159776 A
Patent Literature 2: WO 2016/129444 A

### Summary of Invention

### Technical Problem

Even if the PID disclosed in Patent Literature 1 or 2 is used, a target protein can be labeled and detected with a certain accuracy. However, in order to label and detect the target protein with higher accuracy, there is still room for improvement in terms of improving staining performance.

### Solution to Problem

As a result of intensive studies on the above problems, the present inventor has found that the above problems can be solved because phosphor-integrated nanoparticles for target substance detection obtained by modifying a surface of PID with at least one surface modification molecule selected from the group consisting of a predetermined single-chain antibody and an aptamer have an excellent reaction probability with a target substance, and has completed the present invention. That is, the present invention includes, for example, the following [1] to [8].
[1] Phosphor-integrated nanoparticles for target substance detection obtained by surface-modifying phosphor-integrated nanoparticles with a surface modification molecule, in which the surface modification molecule is at least one kind of surface modification molecule selected from the group consisting of a single-chain antibody containing a heavy chain variable region and an aptamer.
[2] The phosphor-integrated nanoparticles for target substance detection according to [1], in which the single-chain antibody containing a heavy chain variable region is a VHH antibody or scFv.
[3] The phosphor-integrated nanoparticles for target substance detection according to [1] or [2], in which the surface modification molecule has a molecular weight of 5 to 32 kDa.
[4] The phosphor-integrated nanoparticles for target substance detection according to any one of [1] to [3], in which the number of surface modification molecules present in the phosphor-integrated nanoparticles for target substance detection is 0.005 to 0.35 per nm² of a surface of the phosphor-integrated nanoparticles.
[5] The phosphor-integrated nanoparticles for target substance detection according to any one of [1] to [4], in which a target substance of the phosphor-integrated nanoparticles for target substance detection is a cancer marker.
[6] The phosphor-integrated nanoparticles for target substance detection according to any one of [1] to [5], in which a particle serving as a matrix of each of the phosphor-integrated nanoparticles is a silica particle or a resin particle.
[7] A lyophilized preparation obtained by lyophilizing a composition containing the phosphor-integrated nanoparticles for target substance detection according to any one of [1] to [6].
[8] A labeling reagent containing the phosphor-integrated nanoparticles for target substance detection according to any one of [1] to [6].

### Advantageous Effects of Invention

The present invention provides phosphor-integrated nanoparticles for target substance detection having high detection performance.

### Description of Embodiments

<Phosphor-integrated nanoparticles for target substance detection>

Phosphor-integrated nanoparticles for target substance detection of the present invention are obtained by surface-modifying phosphor-integrated nanoparticles with a surface modification molecule, in which the surface modification molecule is at least one kind of surface modification molecule selected from the group consisting of a single-chain antibody containing a heavy chain variable region and an aptamer. Hereinafter, the present invention will be described in detail.

### [Surface modification molecule]

The surface modification molecule in the present invention is at least one kind of surface modification molecule selected from the group consisting of a single-chain antibody containing a heavy chain variable region and an aptamer, and surface-modifies phosphor-integrated nanoparticles described later to constitute the phosphor-integrated nanoparticles for target substance detection of the present invention. The surface modification molecule used in the present invention may be one kind or two or more kinds selected from the group consisting of a single-chain antibody containing a heavy chain variable region and an aptamer. The surface modification molecule is preferably one kind selected from the group consisting of a single-chain antibody containing a heavy chain variable region and an aptamer from a viewpoint of storage stability. Hereinafter, the single-chain antibody containing a heavy chain variable region and the aptamer will be described.

### (1) Single-chain antibody containing heavy chain variable region

The single-chain antibody containing a heavy chain variable region is an antibody formed of a heavy chain and a light chain, or a heavy chain antibody formed only of a heavy chain, the heavy chain antibody containing at least one variable region of the heavy chain, in which the variable regions are bonded to each other with a linker such as a peptide. Examples of the single-chain antibody containing a heavy chain variable region include a VHH antibody and scFv. The VHH antibody is an antibody containing a variable region of a heavy chain antibody derived from an animal belonging to a camelid family such as llama, alpaca, or camel, or a cartilage fish such as shark. scFv is an antibody fragment prepared by bonding a heavy chain variable region and a light chain variable region constituting an antigen-bonding site of an antibody to each other with an arbitrary linker.

The antibody is not particularly limited as long as the antibody can be directly or indirectly bonded to a target substance described later in an immunostaining method. The antibody may be a primary antibody that is specifically bonded to a target substance, a secondary antibody that is bonded to the primary antibody, or a higher-order antibody higher than the secondary antibody, but is preferably the primary antibody or the secondary antibody. The VHH antibody can be prepared by a known method, or a commercially available product can be purchased and used.

### (2) Aptamer

The aptamer is not particularly limited as long as the aptamer can specifically recognize a target substance by a method similar to an immunostaining method used for an antibody. The aptamer may be a primary aptamer that is specifically bonded to a target substance, a secondary aptamer that is specifically bonded to a primary antibody that is bonded to a target substance or the primary aptamer, or a higher-order aptamer higher than the secondary aptamer, but is preferably the primary aptamer or the secondary aptamer.

The aptamers can be roughly classified into a nucleic acid (DNA or RNA) aptamer and a peptide aptamer, but both of these can be used as long as these can specifically recognize a target substance or an antibody or a lower-order aptamer bonded to the target substance and can be bonded thereto. The aptamer is preferably a nucleic acid aptamer from a viewpoint of easy availability of oligonucleic acid. The nucleic acid aptamer and the peptide aptamer can be prepared by a known method, or commercially available products can be purchased and used.

The surface modification molecule has a molecular weight of preferably 5 to 32 kDa, more preferably 10 to 31 kDa, still more preferably 10 to 30 kDa from a viewpoint of preparation and purification efficiency.

As the surface modification molecule, either a single-chain antibody containing a heavy chain variable region or an aptamer can be used, but a VHH antibody or an aptamer is preferable from a viewpoint of sequence terminal modification.

### [Target substance]

The surface modification molecule can be bonded to a specific target substance. Examples of the target substance include a protein such as a polypeptide, an amino acid, a complex thereof with a sugar, a lipid, or the like, Biotin, DNP, DIG, and a hapten such as FITC. Specifically, a cancer marker, a signal transduction substance, a hormone, an antibody present in a living body, an artificial antibody (antibody drug), and an administered drug are preferable, and a protein cancer marker is more preferable. Note that variations of a staining method of a cancer marker or the like in pathological diagnosis also include a method for first causing a probe such as an antibody to react with a cancer marker or the like and then detecting a target substance such as a hapten modified with the probe. Specifically, a genetic cancer marker is also an indirect target substance.

Examples of the protein cancer marker include an immune protein expressed in a cancer cell, a pathway protein expressed in a cancer cell, and a progression protein expressed in a cancer cell. As these cancer-related proteins, various proteins are known. An appropriate protein can be selected according to a purpose of diagnosis or treatment, a mechanism of action of a drug to be used, and the like without being particularly limited. For example, proteins encoded by genes of an immune gene panel, a pathway gene panel, and a progression gene panel included in a cancer-related gene expression panel provided by nCounter correspond to an immune protein, a pathway protein, and a progression protein expressed in a cancer cell, respectively. Mutant proteins corresponding to mutant genes of these genes can also be included in the immune protein, the pathway protein, and the progression protein, respectively.

Examples of the immune protein expressed in a cancer cell include CD40, TL1A, GITR-L, 4-188-L, CX4D-L, CD70, HHLA2, ICOS-L, CD85, CD86, etc. CD80, MHC-II, PDL1, PDL2, VISTA, BTNL2, B7-H3, B7-H4, CD48, HVEM, CD40L, TNFRSF25, GITR, 4-188, OX40, CD27, TMIGD2, ICOS, CD28, TCR, LAG3, CTLA4, PD1, CD244, TIM3, BTLA, CD160, and LIGHT, which are immune checkpoint proteins.

Examples of the pathway protein expressed in a cancer cell include: EGFR (HER1), HER2, HER3, HER4, IGFR, and HGFR, which are cancer cell growth factors or cancer cell growth factor receptors; VEGF-A, VEGF-B, VEGF-C, VEGF-D, VEGF-E, VEGF-R, PIGF-1, and PIGF-2, which are cell surface antigens, vascular growth factors, or vascular growth factor receptors; and interferon, interleukin, G-CSF, M-CSF, EPO, SCF, EGF, FGF, IGF, NGF, PDGF, and TGF, which are cytokines or cytokine receptors.

Examples of the progression protein expressed in a cancer cell include ACTG2, ALDOA, APC, BRMS1, CADM1, CAMK2A, CAMK2B, CAMK2D, CCL5, CD82, CDKN1A, CDKN2A, CHD4, CNN1, CST7, CTSL, CXCR2, YBB, DCC, DENR, DLC1, EGLN2, EGLN3, EIF4E2, EIF4EBP1, ENO1, ENO2, ENO3, ETV4, FGFR4, GSN, HK2, HK3, HKDC1, HLA-DPB1, HUNKIL11, KDM1A, KISS1, LDHA, LIFR, MED23, MET, MGAT5, MAP2K4, MT3, MTA1, MTBP, MTOR, MYCL, MYH11, NDRG1, NF2, NFKB1, NME1, NME4, NOS2, NR4A3, PDK1, PEBP4, PFKFB1, PFKFB4, PGK1, PLAUR, PTTG1 RB1, RORB, SET, SLC2A1, SNRPF, SSTR2, TCEB1, TCEB2, TCF20, TF, TLR4, TNFSF10, TP53, TSHR, MMP, MMP2, MMP10, and HIF1, which are cancer progression markers.

Furthermore, examples of the genetic cancer marker include the following nucleic acid molecules. That is, examples of the genetic cancer marker include a naturally occurring nucleic acid such as DNA or RNA (mRNA, tRNA, miRNA, siRNA, non-cording-RNA, and the like), and an artificial nucleic acid such as peptide nucleic acid (PNA), locked nucleic acid (LNA), or bridged nucleic acid (BNA). Specifically, a marker that detects the following genes can be arbitrarily designed with a nucleic acid probe sequence. That is, examples of a gene related to cancer growth and a response ratio of a molecular target drug include HER2, TOP2A, HER3, EGFR, P53, and MET. Furthermore, examples of a gene known as a cancer-related gene include the following genes. Examples of a tyrosine kinase-related gene include ALK, FLT3, AXL, FLT4 (VEGFR3, DDR1, FMS (CSF1R), DDR2, EGFR (ERBB1), HER4 (ERBB4), EML4-ALK, IGF1R, EPHA1, INSR, EPHA2, IRR (INSRR), EPHA3, KIT, EPHA4, LTK, EPHA5, MER (MERTK), EPHA6, MET, EPHA7, MUSK, EPHA8, NPM1-ALK, EPHB1, PDGFRα (PDGFRA), EPHB2, PDGFRβ (PDGFRB), PD-L1, BMI1, LGR5, EPHB3, RET, EPHB4, RON (MST1R), FGFR1, ROS (ROS1), FGFR2, TIE2 (TEK), FGFR3, TRKA (NTRK1), FGFR4, TRKB (NTRK2), FLT1 (VEGFR1), and TRKC (NTRK3).

Examples of a breast cancer-related gene include ATM, BRCA1, BRCA2, BRCA3, CCND1, E-Cadherin, ERBB2, ETV6, FGFR1, HRAS, KRAS, NRAS, NTRK3, p53, and PTEN. Examples of a carcinoid tumor-related gene include BCL2, BRD4, CCND1, CDKN1A, CDKN2A, CTNNB1, HES1, MAP2, MEN1, NF1, NOTCH1, NUT, RAF, SDHD, and VEGFA. Examples of a colorectal cancer-related gene include APC, MSH6, AXIN2, MYH, BMPR1A, p53, DCC, PMS2, KRAS2 (or Ki-ras), PTEN, MLH1, SMAD4, MSH2, STK11, and MSH6. Examples of a lung cancer-related gene include ALK, PTEN, CCND1, RASSF1A, CDKN2A, RB1, EGFR, RET, EML4, ROS1, KRAS2, TP53, and MYC. Examples of a liver cancer-related gene include Axin1, MALAT1, b-catenin, p16 INK4A, c-ERBB-2, p53, CTNNB1, RB1, Cyclin D1, SMAD2, EGFR, SMAD4, IGFR2, TCF1, and KRAS. Examples of a kidney cancer-related gene include Alpha, PRCC, ASPSCR1, PSF, CLTC, TFE3, p54nrb/NONO, and TFEB. Examples of a thyroid cancer-related gene include AKAP10, NTRK1, AKAP9, RET, BRAF, TFG, ELE1, TPM3, H4/D10S170, and TPR. Examples of an ovarian cancer-related gene include AKT2, MDM2, BCL2, MYC, BRCA1, NCOA4, CDKN2A, p53, ERBB2, PIK3CA, GATA4, RB, HRAS, RET, KRAS, and RNASET2. Examples of a prostate cancer-related gene include AR, KLK3, BRCA2, MYC, CDKN1B, NKX3.1, EZH2, p53, GSTP1, and PTEN. Examples of a bone tumor-related gene include CDH11, COL12A1, CNBP, OMD, COL1A1, THRAP3, COL4A5, and USP6.

The antibody drug is a drug containing an antibody (immunoglobulin) that recognizes an antigen as a main component. The antibody drug used as the target substance may be one that has already been put on the market and is used clinically, or a candidate drug used in a clinical test or a clinical trial. Examples of the antibody drug that has been put on the market include humanized anti-HER2 antibody trastuzumab (Herceptin (registered trademark)), anti-CD30 monoclonal antibody brentuximab, anti-VEGF monoclonal antibody bevasizumab, humanized anti-CD33 antibody gemtuzumab, humanized anti-PD-1 antibody pembrolizumab and nibolumab, and an anti-CTLA-4 ipilimumab monoclonal antibody.

Note that as the artificial nucleic acid, the following nucleic acid drugs can also be considered as the target substance. Examples thereof include decoy, antisense, siRNA, ribozyme, miRNA-mimic, and an aptamer. These can be arbitrarily designed, for example, as a molecule targeting a gene such as PDGF-B, C5, MCP-1, SDF-1, or Hepcidin.

### [Phosphor-integrated nanoparticles]

The phosphor-integrated nanoparticles used in the present invention are not particularly limited, but are preferably, for example, particles containing an organic or inorganic substance as a matrix of the phosphor resin particles, having a structure in which a plurality of particles of a phosphor such as a fluorescent dye is fixed and integrated inside or on a surface of the particles as the matrix, and having a nano-sized diameter of less than 1 µm, in which one particle can emit fluorescence with sufficient brightness. The phosphor-integrated nanoparticles have an average particle size of preferably 40 to 300 nm, more preferably 80 to 150 nm. The phosphor-integrated nanoparticles preferably have a particle size variation coefficient of 5 to 15%. The average particle size and the particle size variation coefficient can be calculated by, for example, observing particles with a scanning electron microscope and measuring the particle sizes of 20 particles.

As the particles serving as a matrix of the phosphor-integrated nanoparticles, particles of a substance that can integrate a phosphor by a physical or chemical bonding force can be used. Specific examples of the particle serving as a matrix of each of the phosphor-integrated nanoparticles include a silica particle and a resin particle.

The phosphor integrated in the phosphor-integrated nanoparticles is not particularly limited, but for example, a known organic phosphor dye molecule and a semiconductor nanoparticle (also referred to as a quantum dot or the like) can be used. Hereinafter, when an organic fluorescent dye is used as a phosphor, the phosphor-integrated nanoparticles are referred to as organic fluorescent dye-integrated nanoparticles, and when an inorganic phosphor is used as a phosphor, the phosphor-integrated nanoparticles are referred to as inorganic phosphor-integrated nanoparticles.

As the phosphor used for the phosphor-integrated nanoparticles, a phosphor that emits fluorescence of a predetermined wavelength (color) can be selected according to an application. When there are two or more kinds of target substances, it is possible to select a combination of phosphors that emit fluorescence of different wavelengths corresponding to the target substances, and to prepare a phosphor-integrated nanoparticles in which each of the phosphors is integrated. In this case, it is preferable to select phosphors whose fluorescence wavelength peaks are separated from each other by 100 nm or more.

### (1) Organic fluorescent dye-integrated nanoparticles

The organic fluorescent dye-integrated nanoparticles are preferably nano-sized fluorescent particles each obtained by integrating a plurality of particles of an organic fluorescent dye inside or on a surface of a substance serving as a matrix of the particles.

Examples of the organic fluorescent dye include a fluorescent dye formed of a low molecular weight organic compound that is not a high molecular weight organic compound including a polymer, such as a rhodamine-based dye, a pyrromethene-based dye, a fluorescein-based dye, an Alexa Fluor (registered trademark, manufactured by Invitrogen)-based dye, a BODIPY (registered trademark, manufactured by Invitrogen)-based dye, a Cascade (registered trademark, manufactured by Invitrogen)-based dye, a coumarin-based dye, an NBD (registered trademark)-based dye, a pyrene-based dye, a cyanine-based dye, a perylene-based dye, or an oxazine-based dye. Among these dyes, a rhodamine-based dye such as TAMRA (registered trademark), sulforhodamine 101, or Texas Red (registered trademark) that is a hydrochloride of sulforhodamine 101, a pyrromethene-based dye such as pyrromethene 556, and a perylene-based dye such as perylene diimide are preferable because of having high light resistance.

Examples of the matrix constituting the organic fluorescent dye-integrated nanoparticles include a substance that can integrate an organic fluorescent dye by a physical or chemical bonding force, such as resin or silica. Specifically, a resin such as polystyrene, polyamide, polylactic acid, polyacrylonitrile, polyglycidyl methacrylate, polymelamine, polyurea, polybenzoguanamine, polyfuran, polyxylene, a phenol resin, or an ASA resin (acrylonitrile-styrene-methyl acrylate copolymer), a polysaccharide, or a hydrophobic compound such as silica is preferable. A melamine resin and a styrene resin are more preferable because fluorescent dye-integrated nanoparticles can be easily prepared and particles having a high luminescence intensity can be obtained.

For example, organic fluorescent dye-integrated nanoparticles prepared by using a fluorescent dye such as TAMRA (registered trademark) or pyrromethene as a phosphor and using a resin such as a melamine resin or silica as a matrix are excellent in function, performance, and the like, and therefore are preferable as the phosphor-integrated nanoparticles.

### (2) Inorganic phosphor-integrated nanoparticles

The inorganic phosphor-integrated nanoparticles are preferably nano-sized fluorescent particles each obtained by integrating a plurality of semiconductor nanoparticles inside or on a surface of a substance serving as a matrix of the particles. Examples of the semiconductor nanoparticles include a quantum dot containing a group II-VI compound, a group III-V compound, or a group IV element, and a particle dot such as CdSe exemplified in WO 2012/133047 A.

In addition, a quantum dot having the semiconductor nanoparticle as a core and having a shell around the semiconductor nanoparticle can also be used. Note that hereinafter, a core-shell type semiconductor nanoparticle is referred to as A/B, in which A represents a core and B represents a shell. Specific examples of the semiconductor nanoparticle having a shell include CdSe/ZnS exemplified in WO 2012/133047 A.

As the semiconductor nanoparticles, semiconductor nanoparticles that have been surface-treated with an organic polymer or the like can be used. Examples of the surface-treated semiconductor nanoparticles include CdSe/ZnS (manufactured by Invitrogen) having a particle surface modified with a carboxyl group and CdSe/ZnS (manufactured by Invitrogen) having a particle surface modified with an amino group.

Examples of the matrix constituting the inorganic phosphor-integrated nanoparticles include a substance that can integrate an inorganic fluorescent dye by a physical or chemical bonding force, such as resin or silica. Specific examples of the resin include: a thermosetting resin such as a melamine resin, a urea resin, a benzoguanamine resin, a phenol resin, or a xylene resin; and various homopolymers and copolymers prepared by using one or more kinds of monomers, such as a styrene resin, a (meth)acrylic resin, polyacrylonitrile, an acrylonitrile-styrene copolymer (AS resin), or an acrylonitrile-styrene-methyl acrylate copolymer (ASA resin).

### [Number of surface modification molecules]

In the phosphor-integrated nanoparticles for target substance detection of the present invention, a considerable number of surface modification molecules are preferably bonded to a surface of one phosphor-integrated nanoparticle. Specifically, the number of surface modification molecules bonded per nm² of the surface of the phosphor-integrated nanoparticle is usually 0.005 to 0.35, preferably 0.009 to 0.32 from a viewpoint of improving staining performance, more preferably 0.038 to 0.32, and still more preferably 0.038 to 0.15.

The number of surface modification molecules bonded per unit area of the surfaces of the phosphor-integrated nanoparticles for target substance detection can be calculated as follows, for example, by applying the method described in WO 2016/129444 A.

The surface modification molecule itself is a protein or a nucleic acid. Therefore, when the surface modification molecule is a protein, by quantifying a protein in a dispersion solution of purified phosphor-integrated nanoparticles using a protein quantification kit based on a BCA method or the like, for example, "Bio-Rad Protein Assay" (manufactured by Bio-Rad Laboratories, Inc.), the total mass (mg) of the surface modification molecules bonded to the surface of the phosphor-integrated nanoparticles can be measured.

Since the molecular weight of the surface modification molecule is known, by dividing the molecular weight of the surface modification molecule by the total mass (mg) of the surface modification molecules, the number of moles of the surface modification molecule bonded to the surface of the phosphor-integrated nanoparticles in the dispersion can be calculated. In addition, the number (M) of the surface modification molecules bonded to the surface of the phosphor-integrated nanoparticles can be calculated from the number of moles and the Avogadro constant.

Meanwhile, the average particle size of the phosphor-integrated nanoparticles can be examined by a known measuring method. Examples thereof include a method for observing the phosphor-integrated nanoparticles using a transmission electron microscope to determine the average particle size as an average particle size of a particle size distribution, a method for measuring a particle size distribution of the particles by a dynamic light scattering method to determine the average particle size as an average particle size of the particle size distribution, and a method for observing the phosphor-integrated nanoparticles using a scanning electron microscope (SEM) to determine the average particle size as an average particle size of a particle size distribution. The surface area (S) of the phosphor-integrated nanoparticles can be calculated from the formula of a surface area of a sphere using a half value of the obtained average particle size of the phosphor-integrated nanoparticles as a radius.

In addition, the number of particles (N) is measured in the dispersion of the phosphor-integrated nanoparticles by means such as a particle counter. From the numerical values obtained above, the number of the surface modification molecules per nm² of the particle surface can be calculated from a formula of M/S/N.

### <Manufacture of phosphor-integrated nanoparticles>

The organic fluorescent dye-integrated nanoparticles and the inorganic phosphor-integrated nanoparticles are known. As for details of a phosphor and a matrix used for manufacturing the organic fluorescent dye-integrated nanoparticles and the inorganic phosphor-integrated nanoparticles, methods for manufacturing the organic fluorescent dye-integrated nanoparticles and the inorganic phosphor-integrated nanoparticles, and the like, and specific examples of embodiments, it is only required to refer to, for example, WO 2013/035703 A, WO 2013/147081 A, or WO 2014/136776 A.

For example, phosphor-encapsulating silica particles containing silica as a matrix and encapsulating a phosphor therein, serving as the phosphor-integrated nanoparticles, can be prepared by dropwise adding a solution containing a phosphor such as an organic fluorescent dye or semiconductor nanoparticles and a silica precursor such as tetraethoxysilane to a solution in which ethanol and ammonia are dissolved, and hydrolyzing the silica precursor.

For example, phosphor-integrated resin particles containing resin as a matrix and having phosphor particles adsorbed on a surface thereof or encapsulating phosphor particles therein, serving as the phosphor-integrated nanoparticles, can be prepared by adding a phosphor such as an organic fluorescent dye or semiconductor nanoparticles to a resin solution or a dispersion of fine particles, and stirring the resulting mixture. Alternatively, the phosphor-integrated resin particles can be prepared by adding a fluorescent dye to a solution of a resin raw material and then accelerating a polymerization reaction.

For example, when phosphor-integrated resin particles are prepared using a thermosetting resin such as a melamine resin as a matrix, the phosphor-integrated resin particles can be prepared by an emulsion polymerization method for heating a mixture containing a prepolymer serving as a raw material of the resin, such as a monomer, an oligomer, or methylol melamine which is a condensate of melamine and formaldehyde, and an organic fluorescent dye, preferably further containing a surfactant and a polymerization reaction accelerator.

### <Manufacture of phosphor-integrated nanoparticles for target substance detection>

The phosphor-integrated nanoparticles for target substance detection are manufactured by surface-modifying phosphor-integrated particles with a surface modification molecule as described above. The method for surface-modifying the phosphor-integrated nanoparticles with the surface modification molecule can be performed by a known method. For example, directly bonding the surface modification molecule and the phosphor-integrated nanoparticle to each other by a covalent bond is preferable because stable phosphor-integrated nanoparticles for target substance detection having a strong bonding force between the surface modification molecule and the phosphor-integrated nanoparticle can be obtained. In particular, a covalent bond using thiol addition to an amide bond, an ester bond, an imide bond, or a maleimide bond is preferable, and a covalent bond using thiol addition to a maleimide bond is more preferable from a viewpoint of a bonding force between the surface modification molecule and the phosphor-integrated nanoparticle.

When phosphor-integrated nanoparticles containing silica as a matrix are used as the phosphor-integrated nanoparticles, a silanol group on a surface of the silica particles may be used as a functional group of the silica particles, or a functional group other than the silanol group, introduced into a surface of the silica particles using a silane coupling agent, for example, an amino group may be used.

Examples of the silane coupling agent used here include a compound having a hydrolyzable group such as an alkoxy group, an acetoxy group, or a halo group at one end and having a functional group such as an amino group, a mercapto group, or an epoxy group at the other end. The hydrolyzable group of the silane coupling agent becomes a silanol group by a hydrolysis reaction and then causes a condensation reaction with a silanol group on a surface of the silica particles (or a silanol group of another silane coupling agent). Therefore, the above functional group such as an amino group can be introduced into the surface of the silica particles.

When phosphor-integrated nanoparticles containing a melamine resin as a matrix are used, by causing the phosphor-integrated nanoparticles to react with a silane coupling agent having a hydrolyzable group (for example, a trialkoxy group) to be adsorbed on a melamine resin at one end and having an amino group at the other end, or causing the phosphor-integrated nanoparticles to react with a linker having amino groups at both ends, an amino group can be introduced into a surface of the melamine resin particles.

A surface of the phosphor-integrated nanoparticles into which an amino group has been introduced is modified with a maleimide group using a known linker having a maleimide group and an NHS group.

In order to introduce a thiol group that reacts with the maleimide group into the surface modification molecule, for example, a disulfide bond of the surface modification molecule is cleaved with a reducing agent such as dithiotreitol or iminothiolane to generate a thiol group in the surface modification molecule.

The above phosphor-integrated nanoparticles having a surface modified with a maleimide group and the above surface modification molecule modified with a thiol group are mixed in a buffer such as PBS and caused to react with each other for a predetermined time to obtain phosphor-integrated nanoparticles for target substance detection, surface-modified with the surface modification molecule.

By lyophilizing a composition containing the phosphor-integrated nanoparticles surface-modified with the surface modification molecule, obtained as described above, that is, the phosphor-integrated nanoparticles for target substance detection, a lyophilized preparation which is another aspect of the present invention can be obtained. Specifically, by lyophilizing a composition obtained by dispersing the phosphor-integrated nanoparticles for target substance detection in a sucrose solution, the lyophilized preparation can be obtained. When conventional phosphor-integrated nanoparticles for target substance detection are lyophilized and then used for staining, detection performance is significantly reduced. However, use of the lyophilized preparation of the present invention for staining is preferable because reduction in detection performance is small.

In addition, by dispersing the phosphor-integrated nanoparticles surface-modified with the surface modification molecule, obtained as described above, that is, the phosphor-integrated nanoparticles for target substance detection in an appropriate solvent preferably after unreacted substances and impurities are removed by a purification treatment using centrifugation or a column, a washing treatment, or the like, a labeling reagent containing the phosphor-integrated nanoparticles for target substance detection, which is another aspect of the present invention, can be obtained. The labeling reagent can not only stain a tissue or a cell by being bonded to a target substance specifically expressed in the tissue or the cell, or a higher-order antibody bonded to the target substance, but can also be used for an analysis method using an antigen-antibody reaction such as an ELISA method or a FACS method.

### Examples

Hereinafter, the present invention will be described more specifically based on Examples, but is not limited to these Examples.

### <Manufacture of phosphor-integrated nanoparticles>

### [Manufacture Example 1] Manufacture of PID-T (TAMRA-encapsulating silica nanoparticles)

By a method including the following steps (1-1) to (1-9), phosphor-integrated nanoparticles (hereinafter, also referred to as "PID-T") which are silica nanoparticles encapsulating TAMRA (registered trademark) (5-carboxytetramethylrhodamine) (hereinafter simply referred to as "TAMRA") which is a fluorescent dye were prepared, and a surface thereof was modified with a maleimide group.

Step (1-1): 2 mg of an N-hydroxysuccinimide ester derivative of TAMRA (TAMRA-NHS ester) and 400 µL (1.796 mmol) of tetraethoxysilane were mixed to prepare a reaction solution.

Step (1-2): Separately from the reaction solution prepared in step (1-1), 40 mL of ethanol and 10 mL of 14% ammonia water were mixed to prepare a mixed solution.

Step (1-3): The reaction solution prepared in step (1-1) was added to the mixed solution prepared in step (1-2) while the mixed solution prepared in step (1-2) was stirred at room temperature. Stirring was performed at room temperature for 12 hours from start of the addition to prepare a mixed reaction product.

Step (1-4): The mixed reaction product prepared in step (1-3) was centrifuged at 10000 g for 60 minutes, and the supernatant was removed. Ethanol was added to the residue to disperse the precipitate, and then centrifugation was performed again under the same conditions as above. Furthermore, with each of ethanol and pure water, washing by dispersion and centrifugation was performed once under similar conditions to obtain TAMRA-encapsulating silica nanoparticles.

By observing the obtained TAMRA-encapsulating silica nanoparticles with a scanning microscope (SEM; S-800 type manufactured by Hitachi, Ltd.), the TAMRA-encapsulating silica nanoparticles had an average particle size of 100 nm and an average particle size variation coefficient of 15%.

Step (1-5): 1 mg of the TAMRA-encapsulating silica nanoparticles obtained in step (1-4) were dispersed in 5 mL of pure water. To the dispersion, 100 µL of 3-aminopropyltriethoxysilane (LS-3150 or KBE-903; manufactured by Shin-Etsu Chemical Co., Ltd.) aqueous dispersion was added, and the resulting mixture was stirred at room temperature for 12 hours to react, thus introducing an amino group into a surface of the TAMRA-content silica nanoparticles.

Step (1-6): The reaction solution was centrifuged at 10000 g for 60 minutes, and the supernatant was removed. Ethanol was added to the residue to disperse the precipitate, and then centrifugation was performed again at 10000 g for 60 minutes. By a similar procedure, washing was performed once with each of ethanol and pure water. When the obtained TAMRA-encapsulating silica nanoparticles modified with an amino group were subjected to FT-IR measurement, spectral absorption derived from the amino group was observed, and it was confirmed that the TAMRA-encapsulating silica nanoparticles were modified with the amino group.

Step (1-7): Using phosphate-buffered saline (PBS) containing 2 mM ethylenediaminetetraacetic acid (EDTA), the TAMRA-encapsulating silica nanoparticles modified with an amino group, obtained in step (1-6) were prepared to 3 nM.

Step (1-8): SM (PEG) 12 (succinimidyl-[(N-maleimidopropionamid)-dodecaethyleneglycol]; manufactured by Thermo Scientific) was mixed with the prepared solution so as to have a final concentration of 10 mM to cause a reaction at room temperature for one hour.

Step (1-9): The reaction solution was centrifuged at 10000 g for 60 minutes, and the supernatant was removed. PBS containing 2 mM EDTA was added thereto to disperse the precipitate, and then centrifugation was performed again at 10000 g for 60 minutes. Dispersion with PBS and washing by centrifugation were performed three times. Finally, the precipitate was dispersed again in 500 µL of PBS to obtain 500 µL of a dispersion of the TAMRA-encapsulating silica nanoparticles modified with a maleimide group. The number of particles was counted using a light scattering type liquid particle counter (manufactured by Rion Co., Ltd. or the like). The particle concentration of the dispersion was measured. PBS was added thereto to prepare a dispersion having a particle concentration of 0.67 nM.

### [Manufacture Example 2] Manufacture of pyrromethene-encapsulating melamine resin nanoparticles (PID-P)

By a method including the following steps (2-1) to (2-5), phosphor-integrated nanoparticles (hereinafter, also referred to as "PID-P") which are silica nanoparticles encapsulating a fluorescent dye Pyrromethene 556 ([[(3,5-dimethyl-4-sulfo-1H-pyrrol-2-yl) (3,5-dimethyl))-4-sulfo-2H-pyrrol-2-ylidene) methyl] methane] (difluoroboran) disodium; manufactured by Tokyo Chemical Industry Co., Ltd.) (hereinafter referred to simply as "pyrromethene") were prepared.

Step (2-1): To a solution obtained by adding 14.4 mg of pyrromethene to 22 mL of water and dissolving the pyrromethene in the water, 2 mL of a 5% aqueous solution of an emulsion polymerization emulsifier Emulgen 430 (polyoxyethylene oleyl ether; manufactured by Kao Corporation) was added.

Step (2-2): The temperature of the solution obtained in step (2-1) was raised to 70°C while the solution was stirred on a hot stirrer. Thereafter, a reaction solution obtained by adding 0.65 g of a melamine resin raw material Nikalac MX-035 (manufactured by Nippon Carbide Industries Co., Ltd.) to the solution was prepared.

Step (2-3): To the reaction solution prepared in step (2-2), 1000 µL of a 10% aqueous solution of dodecylbenzenesulfonic acid (manufactured by Kanto Chemical Co., Inc.) as a reaction initiator was added, and the resulting mixture was heated and stirred at 70°C for 50 minutes to obtain a dispersion of pyrromethene-encapsulating melamine resin nanoparticles.

Step (2-4): The dispersion obtained in step (2-3) was centrifuged at 20000 g for 15 minutes, and the supernatant was removed. Thereafter, ultrapure water was added to the residue, and the resulting mixture was irradiated with ultrasonic waves and redispersed. This washing operation from the centrifugation to the redispersion was performed five times to obtain pyrromethene-encapsulating melamine resin nanoparticles.

By observing the obtained pyrromethene-encapsulating melamine resin nanoparticles with a scanning microscope (SEM; S-800 type manufactured by Hitachi, Ltd.), the pyrromethene-encapsulating silica nanoparticles had an average particle size of 145 nm and an average particle size variation coefficient of 12%. A dispersion having a particle concentration of 0.67 nM was prepared in a similar manner to Manufacture Example 1.

Step (2-5): A maleimide was introduced into a surface of the pyrromethene-encapsulating melamine resin nanoparticles using an NHS-polyethyleneglycol (PEG)-maleimide reagent.

### <Preparation of antibody and aptamer>

An antibody and an aptamer each having a predetermined SH group for surface-modifying the phosphor-integrated nanoparticles were prepared as follows.

### [Preparation Example 1] Preparation of SH group-containing RNA aptamer that recognizes HER2 antigen

A 34 mer RNA sequence (SEQ ID NO: 1) was synthesized by requesting Hokkaido System Science Co., Ltd. to synthesize the 34 mer RNA sequence (SEQ ID NO: 1). The 34 mer RNA sequence (SEQ ID NO: 1) synthesized by Hokkaido System Science Co., Ltd. used phosphoramidite (product number: 10-1936, manufactured by Glen Reserch Co., Ltd.) during synthesis, and had a 5' terminal modified with a disulfide group.

According to the following procedure, the disulfide group was reduced to convert the aptamer into an aptamer having an SH group to obtain an SH group-containing RNA aptamer that recognizes a HER2 antigen.

SEQ ID NO: 1: 5'-AGCCGCGAGGGGAGGGAUAGGGUAGGGCGCGGCU-3'
(1) The dried disulfide aptamer was dissolved in an appropriate amount of a 0.1 M DTT (PromoCell GmbH/PK-CA577-1201-1) solution (pH 8.3 to 8.5).
(2) A reaction was caused at room temperature (about 25°C) for 16 hours.
(3) The reaction solution was caused to pass through a simple oligomer cartridge (Glen Research/Poly-Pac/60-1100-10), and DTT was removed.

### [Preparation Example 2] Preparation of SH group-containing VHH antibody that recognizes HER2 antigen

An anti-HER2-VHH antibody having the following amino acid sequence was prepared as follows. A protein obtained by adding a 15 amino acid (SPSTP-PTPSP-STPPC) linker to a C-terminal of the following amino acid sequence (SEQ ID NO: 2) was prepared as a recombinant. In the obtained performance product, a terminal cysteine formed a dimer by a disulfide bond. Therefore, the performance product was reduced with DTT in a similar manner to [Preparation Example 1] to be converted into an antibody having an SH group, and was used in the following Examples.

SEQ ID NO: 2:

EVQLVESGGGLVQAGGSLRLSCAASGITFSINTMGWYRQAPGKQRELVALISSIGDTYYADSVKGRFTISRD
NAKNTVYLQMNSLKPEDTAVYYCKRFRTAQGTDYWGQGTQVTVSS

[Preparation Example 3] Preparation of SH group-containing scFv antibody that recognizes HER2 antigen

An anti-HER2-scFv antibody was purchased from RANDOX LIFE SCIENCE (product number: RAF9573).

500 µg of the anti-HER2-scFv antibody was dissolved in 200 µL of Borate Buffer containing EDTA. To this solution, 0.05 mL (2.5 × 10⁻³ mol) of 500 mM 2-iminothiolane was added, and the resulting mixture was adjusted to pH 8.5 and then caused to react at room temperature for 60 minutes. The reaction solution was supplied to a gel filtration column, and excess 2-iminothiolane was removed to prepare a solution of an SH group-containing scFv antibody that recognizes a HER2 antigen.

### [Preparation Example 4] Preparation of SH group-containing Fab antibody that recognizes HER2 antigen

By treating an anti-HER2-whole antibody described later with Pierce (trademark) Fab Preparation Kit (manufactured by Thermo Scientific), an anti-HER2-Fab antibody was prepared according to the instructions attached to the product.

An SH group-containing Fab antibody that recognizes a HER2 antigen was prepared in a similar manner to Preparation Example 3 except that the anti-HER2-scFv antibody was replaced with the anti-HER2-Fab antibody prepared above.

[Preparation Example 5] Preparation of SH group-containing F(ab')₂ antibody that recognizes HER2 antigen

By treating an anti-HER2-whole antibody described later with Pierce (trademark) F(ab')₂ Preparation Kit (manufactured by Thermo Scientific), an anti-HER2-F(ab')₂ antibody was prepared according to the instructions attached to the product.

An SH group-containing F(ab')₂ antibody that recognizes a HER2 antigen was prepared in a similar manner to Preparation Example 3 except that the anti-HER2-scFv antibody was replaced with the anti-HER2-F(ab')₂ antibody prepared above.

### [Preparation Example 6] Preparation of SH group-containing whole antibody that recognizes HER2 antigen

An anti-HER2-whole antibody was purchased from Abcam (product code: EP1045Y). An SH group-containing whole antibody that recognizes a HER2 antigen was prepared in a similar manner to Preparation Example 3 except that the anti-HER2-scFv antibody was replaced with the anti-HER2-whole antibody and 2-iminothiolane was replaced with SAT(PEG)4.

### [Preparation Example 7] Preparation of SH group-containing RNA aptamer that recognizes rabbit IgG antibody

A 74 mer RNA sequence (SEQ ID NO: 3) was synthesized by requesting Hokkaido System Science Co., Ltd. to synthesize the 74 mer RNA sequence (SEQ ID NO: 3). The 74 mer RNA sequence (SEQ ID NO: 3) synthesized by Hokkaido System Science Co., Ltd. used phosphoramidite (product number: 10-1936, manufactured by Glen Reserch Co., Ltd.) during synthesis, and had a 5' terminal modified with a disulfide group.

According to the procedure of Preparation Example 1, the disulfide group was reduced to convert the aptamer into an aptamer having an SH group to prepare an SH group-containing RNA aptamer that recognizes a rabbit IgG antibody.

SEQ ID NO: 3: 5'-

GGGAGAAUUCCGACCAGAAGUUCGAUACGCCGUGGGUGACGUUGGCUACCCUUUCCUCUCUCCUC
CUUCUUCU-3'

### [Preparation Example 8] Preparation of SH group-containing VHH antibody that recognizes rabbit IgG antibody

An anti-rabbit IgG-VHH antibody was purchased from Creative Biolabs (product number: CBMAB-003YC). An SH group-containing VHH antibody that recognizes a rabbit IgG antibody was prepared in a similar manner to Preparation Example 3 except that the anti-HER2-scFv antibody was replaced with the anti-rabbit IgG-VHH antibody.

### [Preparation Example 9] Preparation of SH group-containing scFv antibody that recognizes rabbit IgG antibody

An anti-rabbit IgG-scFv antibody was purchased from Creative Biolabs (product number: DrMAB-948). An SH group-containing scFv antibody that recognizes a rabbit IgG antibody was prepared in a similar manner to Preparation Example 3 except that the anti-HER2-scFv antibody was replaced with the anti-rabbit IgG-scFv antibody.

### [Preparation Example 10] Preparation of SH group-containing Fab antibody that recognizes rabbit IgG antibody

An anti-rabbit IgG-Fab antibody was purchased from Abcam (product number: ab6824). An SH group-containing Fab antibody that recognizes a rabbit IgG antibody was prepared in a similar manner to Preparation Example 3 except that the anti-HER2-scFv antibody was replaced with the anti-rabbit IgG-Fab antibody.

### [Preparation Example 11] Preparation of SH group-containing F(ab')₂ antibody that recognizes rabbit IgG antibody

An anti-rabbit IgG-Fab antibody was purchased from Abcam (product code: ab98437). An SH group-containing F(ab')₂ antibody that recognizes a rabbit IgG antibody was prepared in a similar manner to Preparation Example 3 except that the anti-HER2-scFv antibody was replaced with the anti-rabbit IgG-F(ab')₂ antibody.

[Preparation Example 12] Preparation of SH group-containing half antibody that recognizes rabbit IgG antibody

An anti-rabbit IgG-whole antibody was purchased from SynAbs (product number: LO-RG1). According to the following protocol of Dojindo Kit (LK10), an SH group-containing half antibody that recognizes a rabbit IgG antibody was prepared by a reduction method using a reducing agent.
(1) A required amount (maximum 30 µL) of Reaction Buffer was put in a microtube, and an equal amount of DMSO was added thereto to prepare a Working buffer.
(2) An antibody solution prepared to 0.5 to 1 mg/mL, in which the amount of an antibody corresponded to 10 µg, was put in another microtube.
(3) The Working buffer prepared in (1) was added to the antibody solution of (2) and mixed therewith by pipetting.

### [Preparation Example 13] Preparation of SH group-containing RNA aptamer that recognizes FITC hapten

A 90 mer RNA sequence (SEQ ID NO: 4) was synthesized by requesting Gene Design to synthesize the 90 mer RNA sequence (SEQ ID NO: 4). The 90 mer RNA sequence (SEQ ID NO: 4) synthesized by Gene Design used phosphoramidite (product number: 10-1936, manufactured by Glen Reserch Co., Ltd.) during synthesis, and had a 5' terminal modified with a disulfide group.

According to the procedure of Preparation Example 1, the disulfide group was reduced to convert the aptamer into an aptamer having an SH group to prepare an SH group-containing RNA aptamer that recognizes FITC hapten.

SEQ ID NO: 4: 5'-

GGACGGCACCACGGUCGGAUCCGUGAGUUGUGACAAUUUAGCGGGUGGUAUUAGAGCCUACUGCC
ACAGCAAUAGGAUCGAUACAGAUCU-3'

### [Preparation Example 14] Preparation of SH group-containing Fab antibody that recognizes FITC hapten

By treating an anti-FITC-whole antibody described later with Pierce (trademark) Fab Preparation Kit (manufactured by Thermo Scientific), an anti-FITC-Fab antibody was prepared according to the instructions attached to the product.

An SH group-containing Fab antibody that recognizes FITC hapten was prepared in a similar manner to Preparation Example 3 except that the anti-HER2-scFv antibody was replaced with the anti-FITC-whole antibody prepared above.

[Preparation Example 15] Preparation of SH group-containing F(ab')₂ antibody that recognizes FITC hapten

By treating an anti-FITC-whole antibody described later with Pierce (trademark) F(ab')₂ Preparation Kit (manufactured by Thermo Scientific), an anti-FITC-F(ab')₂ antibody was prepared according to the instructions attached to the product.

An SH group-containing F(ab')₂ antibody that recognizes FITC hapten was prepared in a similar manner to Preparation Example 3 except that the anti-HER2-scFv antibody was replaced with the anti-FITC-F(ab')₂ antibody prepared above.

### [Preparation Example 16] Preparation of SH group-containing whole antibody that recognizes FITC hapten

An anti-FITC-whole antibody was purchased from Bethyl Laboratories (product number: A150-112A). An SH group-containing whole antibody that recognizes FITC hapten was prepared in a similar manner to Preparation Example 3 except that the anti-HER2-scFv antibody was replaced with the anti-FITC-whole antibody.

### <Manufacture of surface-modified phosphor-integrated nanoparticles for target substance detection>

Using the PID particles manufactured in Manufacture Examples 1 and 2 and the antibodies and aptamers prepared in Preparation Examples 1 to 16, surface-modified phosphor-integrated nanoparticles for target substance detection were prepared as follows.

### [Example 1]

740 µL of the PID-T (0.67 nM) modified with a maleimide group manufactured in Manufacture Example 1 and 50 µg of the SH group-containing RNA aptamer that recognizes a HER2 antigen prepared in Preparation Example 1 were mixed in 1 mL of EDTA-containing phosphate Buffer, and a reaction to bond both molecules to each other was caused at room temperature for one hour.

The reaction solution was centrifuged at 10000g for 60 minutes, and the supernatant was removed. PBS containing 2 mM EDTA was added thereto to disperse the precipitate, and then centrifugation was performed again at 10000 g for 60 minutes. Dispersion with PBS and washing by centrifugation were performed three times. Finally, 500 µL of PBS was added to obtain PID-T surface-modified with a HER2 antigen-recognizing RNA aptamer.

### [Example 2]

PID-T surface-modified with an anti-HER2-VHH antibody was obtained in a similar manner to Example 1 except that the SH group-containing RNA aptamer that recognizes a HER2 antigen was replaced with the SH group-containing VHH antibody that recognizes a HER2 antigen prepared in Preparation Example 2.

### [Example 3]

PID-T surface-modified with an anti-HER2-scFv antibody was obtained in a similar manner to Example 1 except that the SH group-containing RNA aptamer that recognizes a HER2 antigen was replaced with the SH group-containing scFv antibody that recognizes a HER2 antigen prepared in Preparation Example 3.

### [Comparative Example 1]

PID-T surface-modified with an anti-HER2-Fab antibody was obtained in a similar manner to Example 1 except that the SH group-containing RNA aptamer that recognizes a HER2 antigen was replaced with the SH group-containing Fab antibody that recognizes a HER2 antigen prepared in Preparation Example 4.

### [Comparative Example 2]

PID-T surface-modified with an anti-HER2-F(ab')₂ antibody was obtained in a similar manner to Example 1 except that the SH group-containing RNA aptamer that recognizes a HER2 antigen was replaced with the SH group-containing F(ab')₂ antibody that recognizes a HER2 antigen prepared in Preparation Example 5.

### [Comparative Example 3]

PID-T surface-modified with an anti-HER2-whole antibody was obtained in a similar manner to Example 1 except that the SH group-containing RNA aptamer that recognizes a HER2 antigen was replaced with the SH group-containing whole antibody that recognizes a HER2 antigen prepared in Preparation Example 6.

### [Example 4]

PID-P surface-modified with a rabbit IgG antigen-recognizing RNA aptamer was obtained in a similar manner to Example 1 except that the SH group-containing RNA aptamer that recognizes a HER2 antigen was replaced with the SH group-containing RNA aptamer that recognizes a rabbit IgG antibody prepared in Preparation Example 7 and that PID-T was replaced with PID-P.

### [Example 5]

PID-P surface-modified with a rabbit IgG VHH antibody was obtained in a similar manner to Example 1 except that the SH group-containing RNA aptamer that recognizes a HER2 antigen was replaced with the SH group-containing VHH antibody that recognizes a rabbit IgG antibody prepared in Preparation Example 8 and that PID-T was replaced with PID-P.

### [Example 6]

PID-P surface-modified with an anti-rabbit IgG-scFv antibody was obtained in a similar manner to Example 5 except that the SH group-containing VHH antibody that recognizes a rabbit IgG antibody was replaced with the SH group-containing scFv antibody that recognizes a rabbit IgG antibody prepared in Preparation Example 9.

### [Comparative Example 4]

PID-P surface-modified with an anti-rabbit IgG-Fab antibody was obtained in a similar manner to Example 5 except that the SH group-containing VHH antibody that recognizes a rabbit IgG antibody was replaced with the SH group-containing Fab antibody that recognizes a rabbit IgG antibody prepared in Preparation Example 10.

### [Comparative Example 5]

PID-P surface-modified with an anti-rabbit IgG-F(ab')₂ antibody was obtained in a similar manner to Example 5 except that the SH group-containing VHH antibody that recognizes a rabbit IgG antibody was replaced with the SH group-containing F(ab')₂ antibody that recognizes a rabbit IgG antibody prepared in Preparation Example 11.

### [Comparative Example 6]

PID-P surface-modified with an anti-rabbit IgG-half antibody was obtained in a similar manner to Example 5 except that the SH group-containing VHH antibody that recognizes a rabbit IgG antibody was replaced with the rabbit IgG-half antibody prepared in Preparation Example 12.

### [Example 7]

PID-T surface-modified with a FITC antigen-recognizing RNA aptamer was obtained in a similar manner to Example 1 except that the SH group-containing RNA aptamer that recognizes a HER2 antigen was replaced with the SH group-containing RNA aptamer that recognizes FITC hapten prepared in Preparation Example 13.

### [Comparative Example 7]

PID-T surface-modified with an anti-FITC-Fab antibody was obtained in a similar manner to Example 1 except that the SH group-containing RNA aptamer that recognizes a HER2 antigen was replaced with the SH group-containing Fab antibody that recognizes FITC hapten prepared in Preparation Example 14.

### [Comparative Example 8]

PID-T surface-modified with an anti-FITC-F(ab')₂ antibody was obtained in a similar manner to Example 1 except that the SH group-containing RNA aptamer that recognizes a HER2 antigen was replaced with the SH group-containing F(ab')₂ antibody that recognizes FITC hapten prepared in Preparation Example 15.

### [Comparative Example 9]

PID-T surface-modified with an anti-FITC-whole antibody was obtained in a similar manner to Example 1 except that the SH group-containing RNA aptamer that recognizes a HER2 antigen was replaced with the SH group-containing whole antibody that recognizes FITC hapten prepared in Preparation Example 16.

### <Performance evaluation>

### [Staining performance evaluation 1] Bright spot measurement of phosphor-integrated nanoparticles surface-modified with anti-HER2 antibody or the like

Staining performance (sensitivity) of the phosphor-integrated nanoparticles prepared in each of Examples 1 to 3 and Comparative Examples 1 to 3 was evaluated as follows using a positive control slide (product number: 170911-2) of a HER2 gene high expression cell line SKBR3 manufactured by Pathology Institute.

### (1) Deparaffinization treatment

By immersing the specimen slide in xylene for 30 minutes, paraffin in a section was removed and replaced with xylene. By immersing the specimen slide in ethanol for 30 minutes, xylene in the section was replaced with ethanol. By immersing the specimen slide in distilled water for 30 minutes, ethanol was replaced with distilled water.

### (2) Activation treatment

The specimen slide after the treatment of (1) was immersed in 10 mM citrate buffer (pH 6.0) for 30 minutes, and water in the section was replaced with the citrate buffer. The specimen slide was autoclaved (121°C, 10 minutes) and then immersed in PBS for 30 minutes. PBS containing 1% by mass of BSA was added dropwise to the entire section of the specimen slide to be subjected to a blocking treatment at room temperature for one hour.

### (3) Immunostaining treatment

From the surface-modified phosphor-integrated nanoparticles prepared in each of Examples 1 to 3 and Comparative Examples 1 to 3, a 0.05 nM dispersion was prepared using PBS containing 1% by mass of BSA. The dispersion was added dropwise to the entire section of the specimen slide after the treatment of (2) and left at room temperature for three hours.

### (4) Morphological observation treatment

The specimen slide after the treatment of (3) was immersed in PBS for 30 minutes, and then the section was fixed with a 4% paraformaldehyde solution for 10 minutes. The section was stained with a Meyer hematoxylin solution for five minutes, washed with running water at 45°C for three minutes, and then stained with a 1% eosin solution for five minutes to perform hematoxylin-eosin staining. Thereafter, an operation of immersing the section in pure ethanol for five minutes was performed, and washing and dehydration were performed. Subsequently, the section was immersed in xylene for five minutes to be subjected to a permeabilization treatment. Aquatex (registered trademark) (product number: 108562) manufactured by Merck Millipore was added dropwise to the entire section of the specimen slide, and a cover glass was placed on the section and left at room temperature for 30 minutes or more to seal the section, thus preparing an observation specimen slide.

### (5) Bright spot measurement

The section on the observation specimen slide was irradiated with excitation light of 579.5 to 590.5 nm for passing through a bandpass filter (FF01-585/11-25) manufactured by Semrock. Fluorescence of 612 to 644 nm emitted from the surface-modified phosphor-integrated nanoparticles (PID-T) on the section for passing through the bandpass filter (FF01-628/32-25) manufactured by Semrock was observed and imaged with a fluorescence microscope (BX-53, manufactured by Olympus Corporation). Bright spot measurement was performed by an ImageJ Find Maxima method.

During microscopic observation and image acquisition, irradiation energy at the center of the field of view was set to 900 W/cm² at a wavelength of 550 nm. Exposure time at the time of image acquisition was set such that the brightness of an image was not saturated. Using a microscope image imaged with a fluorescence microscope or the like, a portion where the brightness exceeded a predetermined threshold was measured as a bright spot, and the number of bright spots per cell was calculated. Table 1 illustrates results thereof.

### [Staining performance evaluation 2] Bright spot measurement of phosphor-integrated nanoparticles surface-modified with anti-rabbit IgG antibody or the like

Staining performance (sensitivity) of the phosphor-integrated nanoparticles prepared in each of Examples 4 to 6 and Comparative Examples 4 to 6 was evaluated as follows using a specimen slide prepared from a paraffin block of a human lung cancer cell line NCI-H446.

### (1) Preparation of NCI-H446 specimen slide

NCI-H446 was purchased from ATCC and cultured with an RPMI-1640/sigma + 10% FBS/Gibco + 1% L-glutamine/sigma + 1% Pen Strep/Nakarai medium using a CO₂ incubator (Panasonic, MCO-5AC-PJ)) under conditions of 37°C and 5% CO₂. After being cultured, the cell line was subjected to formalin fixation, a dehydration treatment with alcohol, and a xylene treatment according to a conventional method, and was immersed in high-temperature paraffin and embedded in a paraffin to prepare a paraffin block of the cell line. A section having a thickness of 4 µm was cut out from the paraffin block to prepare a specimen slide.

### (2) Pretreatment of specimen slide

The specimen slide was subjected to similar treatments to (1) deparaffinization treatment and (2) activation treatment of the above [Staining performance evaluation 1].

### (3) Primary immunostaining

A reaction solution for primary immunostaining was prepared such that an anti-PD-Ll rabbit monoclonal antibody (clone "SP142"; manufactured by Spring Bioscience) had a concentration of 0.05 nM using PBS containing 1% by mass of BSA. The specimen slide that had been subjected to the above pretreatment of (2) was immersed in the reaction solution for primary immunostaining and was caused to react at 4°C overnight.

### (4) Secondary staining treatment

From the phosphor-integrated nanoparticles prepared in each of Examples 4 to 6 and Comparative Examples 4 to 6, a 0.05 nM dispersion was prepared using PBS containing 1% by mass of BSA. The dispersion was added dropwise to the entire section of the specimen slide after the primary staining treatment of (4) and left at room temperature for three hours.

### (5) Bright spot measurement

A similar treatment to (4) morphological observation treatment in the above [Staining performance evaluation 1] was performed to prepare an observation specimen slide. Using the observation specimen slide of the surface-modified phosphor-integrated nanoparticles prepared in Example 4, a bright spot was measured in a similar manner to (5) of the above [Staining performance evaluation 1], and the number of bright spots per cell was calculated. Table 1 illustrates results thereof. The section on the observation specimen slide of the surface-modified phosphor-integrated nanoparticles prepared in each of Examples 5 and 6 and Comparative Examples 4 to 6 was irradiated with excitation light of 420 to 520 nm for passing through a bandpass filter (FF02-470/100-25) manufactured by Semrock. Fluorescence of 517.5 to 532.5 nm emitted from the phosphor-integrated nanoparticles for target substance detection (PID-P) on the section for passing through the bandpass filter (FF01-525/15-25) manufactured by Semrock was observed and imaged with a fluorescence microscope (BX-53, manufactured by Olympus Corporation). Bright spot measurement was performed by an ImageJ Find Maxima method, and the number of bright spots per cell was calculated.

### [Staining performance evaluation 3] Bright spot measurement of phosphor-integrated nanoparticles surface-modified with anti-FITC antibody or the like

Using a FITC labeling kit (Fluorescein Labeling kit-NH2/LK02: manufactured by Dojindo Molecular Technologies, Inc.), an anti-HER2 antibody (product code: EP1045Y) purchased from Abcam was caused to react with a FITC reagent (BP-22401: manufactured by Broad Pharm) according to the instructions of the kit to prepare a FITC-labeled anti-HER2 antibody.

From the prepared FITC-labeled anti-HER2 antibody, a 0.05 nM FITC-labeled anti-HER2 antibody solution was prepared using PBS containing 1% BSA. The solution was added dropwise to the section on the positive control slide of the HER2 gene high expression cell line SKBR3 that had been subjected to (1) deparaffinization treatment and (2) activation treatment in the above [Staining performance evaluation 1], and caused to react at 4°C overnight to perform a primary immunostaining treatment.

The specimen slide that had been subjected to the above primary immunotreatment was washed with PBS. Thereafter, the dispersion of the phosphor-integrated nanoparticles prepared so as to have a concentration of 0.05 nM using PBS containing 1% by mass of BSA in each of Example 7 and Comparative Examples 7 to 9 was added dropwise to the entire section of the specimen slide after the primary staining treatment and caused to react at room temperature for three hours to perform secondary immunostaining.

The specimen slide that had been subjected to secondary immunostaining was subjected to similar treatment to (4) morphological observation treatment in the above [Staining performance evaluation 1] to prepare an observation specimen slide. Using the observation specimen slide of the surface-modified phosphor-integrated nanoparticles prepared in Example 4, a bright spot was measured in a similar manner to (5) of the above [Staining performance evaluation 1], and the number of bright spots per cell was calculated. Table 1 illustrates results thereof.

**[Table 1]**

| Target substance | Example or Comparative Example | Antibody or Aptamer | Molecular weight (kDa) | Number of bright spots (spot) |
|---|---|---|---|---|
| HER2 | Example 1 | Aptamer | 11 | 251 |
| | Example 2 | VHH | 13 | 288 |
| | Example 3 | scFv | About 30 | 240 |
| | Comparative Example 1 | Fab | About 50 | 148 |
| | Comparative Example 2 | F(ab')₂ | About 100 | 145 |
| | Comparative Example 3 | whole | 137 | 160 |
| Rabbit IgG | Example 4 | Aptamer | 23 | 605 |
| | Example 5 | VHH | About 15 | 702 |
| | Example 6 | scFv | About 30 | 680 |
| | Comparative Example 4 | Fab | About 50 | 452 |
| | Comparative Example 5 | F(ab')₂ | About 100 | 410 |
| | Comparative Example 6 | half | About 75 | 374 |
| FITC | Example 7 | Aptamer | 29 | 81 |
| | Comparative Example 7 | Fab | About 50 | 35 |
| | Comparative Example 8 | F(ab')₂ | About 100 | 44 |
| | Comparative Example 9 | whole | About 150 | 43 |

### <Lyophilizing treatment>

### [Example 8]

To the dispersion of the phosphor-integrated nanoparticles prepared in each of Examples 1 and 2 and Comparative Examples 1 to 3, 5% sucrose was added, and a lyophilizing treatment was performed using a centrifugal evaporator (CVE-200D) under reduced pressure conditions of 0.2 mmHg (28 Pa) to obtain lyophilized powder (lyophilized preparation) of the phosphor-integrated nanoparticles.

### [Particle stability evaluation]

Using a plate-reader (Envision: manufactured by PerkinElmer), stability of the phosphor-integrated nanoparticles of each of Examples 1 and 2 and Comparative Example 3 before and after the lyophilizing treatment was evaluated based on a relative value of a fluorescence intensity of the particles alone at excitation light of 550 nm. For the powder after the lyophilizing treatment, a 0.05 nM dispersion was prepared using PBS containing 1% by mass of BSA. Table 2 illustrates results of the fluorescence intensity.

**[Table 2]**

| Target substance | Antibody or Aptamer | Fluorescence intensity | |
|---|---|---|---|
| | | Before lyophilizing | After lyophilizing |
| HER2 | Aptamer | 100 | 95 |
| | VHH | 100 | 95 |
| | whole | 100 | 60 |

### [Staining performance evaluation 4]

Using the particle dispersion of the phosphor-integrated nanoparticles prepared in each of Examples 1 to 3 and Comparative Examples 1 and 3 before and after the lyophilizing treatment, the number of bright spots was measured by a similar operation to [Staining performance evaluation 1] using the positive control slide of the HER2 gene high expression cell line SKBR3. Table 3 illustrates results thereof. It was observed that the particles modified with the Fab antibody and the whole antibody deteriorated to reduce their performance. It is considered that a smaller protein has a smaller number of water molecules contained in a molecule thereof and is less affected by freezing.

**[Table 3]**

| Target substance | Antibody or Aptamer | Number of bright spots (spot) | |
|---|---|---|---|
| | | Before lyophilizing | After lyophilizing |
| HER2 | Aptamer | 251 | 255 |
| | VHH | 288 | 288 |
| | scFv | 240 | 225 |
| | Fab | 148 | 21 |
| | whole | 160 | 30 |

### <ELISA analysis>

### [Preparation Example 17] SH group-containing DNA aptamer that recognizes PDL1 antigen

An SH group-containing DNA aptamer that recognizes a PDL1 antigen was prepared in a similar manner to Preparation Example 1 except that the 34 mer RNA sequence (SEQ ID NO: 1) of Preparation Example 1 was replaced with a 45 mer DNA sequence (SEQ ID NO: 5).

### SEQ ID NO: 5: 5'-ACGGGCCACATCAACTCATTGATAGACAATGCGTCCACTGCCCGT-3'

### [Example 9]

In Example 1, PID-T surface-modified with a PDL1 antigen-recognizing DNA aptamer was obtained in a similar manner to Example 1 except that the RNA aptamer of Preparation Example 1 was replaced with the SH group-containing DNA aptamer that recognizes a PDL1 antigen of Preparation Example 17.

### [Preparation Example 18]

Preparation of SH group-containing Recombinant antibody that recognizes PDL1 antigen An SH group-containing Recombinant antibody that recognizes a PDL1 antigen was prepared in a similar manner to Preparation Example 6 except that the anti-HER2-whole antibody was replaced with an anti-PDLl-recombinant antibody (clone E1L3N/Cell Signaling Technology/#13684/about 60 kDa).

### [Comparative Example 10]

PID-P surface-modified with an anti-PDL1 antibody was obtained in a similar manner to Example 6 except that the whole antibody that recognizes a HER2 antigen of Preparation Example 6 was replaced with a Recombinant antibody that recognizes a PDL1 antigen of Preparation Example 18.

### [Example 10]

(1) To a HER2 96-well plate, 5 µg/mL of an anti-HER2 antibody (clone: 6C2/GTX82764/GeneTex) was added at 50 µL/well, and incubation was performed at 0°C for 16 hours. The additive solution was aspirated, and 100 µL of 1% BSA/PBS was added and blocked at room temperature for two hours. Thereafter, the blocking solution was aspirated.
   Next, Recombinant human HER2 protein (E27-11G-10/Signal Chem) was diluted with 1% BSA/PBS to 2 ng/mL, and the diluted solution was added at 100 µL/well. Incubation was performed at room temperature for one hour. A washing operation of aspirating the additive solution, adding 200 µL of 0.05% Tween/PBS (PBST), and allowing the solution to stand for five minutes was repeated three times.
   Finally, 0.67 nM of the particle dispersion of the phosphor-integrated nanoparticles prepared in each of Examples 1 and 2 and Comparative Examples 1 to 3 before and after the lyophilizing treatment was added in an amount of 50 µL, and incubation was performed at room temperature for one hour. A washing operation of aspirating the additive solution, adding 200 µL of 0.05% Tween/PBS (PBST), and allowing the solution to stand for five minutes was repeated three times. Thereafter, the 96 well plate was set on a ThermoFishe/Envision plate-reader, and a fluorescence intensity was measured. Table 4 illustrates results thereof.
(2) A fluorescence intensity was measured by replacing the PDL1 anti-HER2 antibody with an anti-PDL 1 antibody (clone 28-8/abcam/ab205921), replacing Recombinant human HER2 protein with Recombinant human PDL1 protein (Bio Vision/7429-50), and replacing the particle dispersion with the particle dispersion of the photon-integrated nanoparticles prepared in each of Example 9 and Comparative Example 10 before and after the lyophilizing treatment. Table 4 illustrates results thereof.

**[Table 4]**

| Target substance | Antibody or Aptamer | Molecular weight (kDa) | Fluorescence intensity (PFU) | |
|---|---|---|---|---|
| | | | Before lyophilizing | After lyophilizing |
| HER2 | Aptamer | 11 | 52000 | 52000 |
| | VHH | 13 | 51000 | 49000 |
| | scFv | About 30 | 52000 | 50000 |
| | Fab | About 50 | 41000 | 18000 |
| | whole | 137 | 33000 | 15000 |
| PDL1 | Aptamer | 14 | 20500 | 19000 |
| | Recombinant | About 60 | 17000 | 5500 |

### <Detection of genetic marker>

### [Example 11]

[Staining performance evaluation 5] Bright spot measurement of phosphor-integrated nanoparticles surface-modified with FITC hapten

The (1) deparaffinization treatment of [Staining performance evaluation 1] was performed, and then the following treatment was performed.

### (Pretreatment of specimen slide)

A pretreatment was performed in the following order of (1) to (6) to remove proteins from a cell membrane and a nuclear membrane.

(1) A slide was treated with 0.2 M HCl at room temperature for 20 minutes. (2) The specimen slide was immersed in purified water for three minutes. (3) The specimen slide was immersed in a washing buffer (2 × SSC: standard sailine citrate) for three minutes. (4) The specimen slide was immersed in a pretreatment solution (1 N NaSCN) at 80°C for 30 minutes. (5) The specimen slide was immersed in purified water for one minute. (6) The specimen slide was immersed in a washing buffer (2 × SSC) for five minutes, and the same operation was repeated twice.

### (Enzyme treatment)

The pretreated specimen slide was subjected to the following treatments (1) and (2) in this order to be subjected to an enzyme treatment. (1) The specimen slide was immersed in a protease solution heated to 37°C for 10 to 60 minutes. This immersion treatment was performed with 25 mg of protease (2500 to 3000 Units/mg) [pepsin]/1 M NaCl [pH 2.0] 50 mL at 37°C for 60 minutes). (2) The specimen slide was immersed in a washing buffer for five minutes. This operation was repeated twice.

### (Preparation of probe)

From a solution of DNA probe (GC006) that recognizes a FITC-labeled HER2 gene sequence purchased from GSP, 2 µL was extracted and mixed with 8 µL of a denaturing solution (70% formamide/SSC [150 mM NaCl, 15 mM sodium citrate]).

### (Preparation of staining specimen slide: denaturation of target gene)

The specimen slide was subjected to the following treatments (1) to (6). (1) A wet box (airtight container with its side sealed with a paper towel) having a moistened paper towel laid on a bottom thereof before preparing a specimen slide was put in a 37°C incubator and preheated. (2) It was confirmed that the pH of a denaturing solution (70% formamide/SSC [150 mM NaCl, 15 mM sodium citrate]) was pH 7.0 to 8.0 at normal temperature. The denaturing solution was put in a Coplin jar and heated (72 ± 1°C) in a warm bath until the temperature of the solution was 72°C ± 1°C, and put in the warm bath for at least 30 minutes. (3) A hybridization area on the back of the specimen slide was marked with a diamond pen or the like such that the hybridization area was identified. (4) The specimen slide was immersed in a Coplin jar containing a denaturing solution at 72 ± 1°C to denature DNA of the specimen slide. (5) The specimen slide was taken out from the denaturing solution using tweezers and immediately put in 70% ethanol at normal temperature. The specimen slide was shaken in order to remove formamide. The specimen slide was immersed for one minute. (6) The specimen slide was taken out from 70% ethanol, put in 85% ethanol, and shaken in order to remove formamide. The specimen slide was immersed for one minute. The same operation was repeated twice with 100% ethanol.

### (Hybridization)

The denatured specimen slide was subjected to the following treatments (1) to (3) in this order, and was thereby subjected to a hybridization treatment using 10 µL (10 to 50 ng) of the DNA probe prepared as described above. (1) 10 µL of the prepared DNA probe was added to a hybridization area of the specimen slide, and immediately the probe was covered with a 22 mm × 22 mm cover glass to spread the probe uniformly. At this time, air bubbles were prevented from entering the hybridization area. (2) The cover glass is sealed with paper bond. (3) The specimen slide was put in a preheated wet box, covered with a lid, and was hybridized in a 37°C incubator for 14 to 18 hours.

### (Washing of slide glass)

The hybridized specimen slide was subjected to the following treatments (1) to (5) in this order, and was thereby washed. (1) A post-hybridization washing buffer (2 × SSC/0.3% NP-40) was put in a Coplin jar. The post-hybridization washing buffer was preheated to 72°C ± 1°C in a warm bath (put in a warm bath at 72°C ± 1°C for at least 30 minutes). (2) Another Coplin jar containing the post-hybridization washing buffer was prepared and maintained at normal temperature. (3) The paper bond sealing was removed with tweezers. (4) The specimen slide was put in the post-hybridization washing buffer. The cover glass was left until the cover glass was naturally peeled off in the solution. (5) The specimen slide was taken out from the solution, an excess solution was removed, and the specimen slide was immersed in the post-hybridization washing buffer heated to 72 ± 1°C for two minutes. Here, desirably, a temperature exceeding 73°C was maintained for two minutes or less, and treatment was performed for two minutes or less.

### (Immunostaining)

FITC of the hybridized DNA probe that recognizes a FITC-labeled HER2 gene sequence was immunostained using particles that recognize FITC (particles described in Example 7 and Comparative Example 9) in a similar manner to the treatment of (3) in [Staining performance evaluation 1].

### (Bright spot measurement)

After immunostaining, a similar treatment to (4) morphological observation treatment in [Staining performance evaluation 1] was performed, and then bright spot measurement of (5) was performed similarly. As a result of bright spot measurement, 45 red bright spots were confirmed when the particles of Example 7 were used, and 35 red bright spots were confirmed when the particles of Comparative Example 9 were used. From the above, it has been clarified that the present particles can also be used for gene detection. Note that in the bright spot measurement, a red bright spot was measured according to [Staining performance evaluation 1].

SEQ ID NO: 1: RNA base sequence for preparing RNA aptamer
SEQ ID NO: 2: Amino acid sequence for preparing VHH antibody
SEQ ID NO: 3: RNA base sequence for preparing RNA aptamer
SEQ ID NO: 4: RNA base sequence for preparing RNA aptamer
SEQ ID NO: 5: DNA base sequence for preparing DNA aptamer

## Claims

1. Phosphor-integrated nanoparticles for target substance detection obtained by surface-modifying phosphor-integrated nanoparticles with a surface modification molecule, wherein
the surface modification molecule is at least one kind of surface modification molecule selected from the group consisting of a single-chain antibody containing a heavy chain variable region and an aptamer.

2. The phosphor-integrated nanoparticles for target substance detection according to claim 1, wherein the single-chain antibody containing a heavy chain variable region is a VHH antibody or scFv.

3. The phosphor-integrated nanoparticles for target substance detection according to claim 1 or 2, wherein the surface modification molecule has a molecular weight of 5 to 32 kDa.

4. The phosphor-integrated nanoparticles for target substance detection according to any one of claims 1 to 3, wherein the number of surface modification molecules present in the phosphor-integrated nanoparticles for target substance detection is 0.005 to 0.35 per nm² of a surface of the phosphor-integrated nanoparticles.

5. The phosphor-integrated nanoparticles for target substance detection according to any one of claims 1 to 4, wherein a target substance of the phosphor-integrated nanoparticles for target substance detection is a cancer marker.

6. The phosphor-integrated nanoparticles for target substance detection according to any one of claims 1 to 5, wherein a particle serving as a matrix of each of the phosphor-integrated nanoparticles is a silica particle or a resin particle.

7. A lyophilized preparation obtained by lyophilizing a composition containing the phosphor-integrated nanoparticles for target substance detection according to any one of claims 1 to 6.

8. A labeling reagent comprising the phosphor-integrated nanoparticles for target substance detection according to any one of claims 1 to 6.


SEQUENCE LISTING

<110>  KONICAMINOLTA. INC

<120>  Phosper Integrated Dots nanoparticles for Detecting Target Substance

<130>  B48669JP01

<160>  5

<170>  PatentIn version 3.5

<210>  1
<211>  34
<212>  RNA
<213>  Artificial Sequence

<220>
<223>  For RNA aptamer

<220>
<221>  misc_feature
<222>  (1)..(34)

<400>  1
agccgcgagg ggagggauag gguagggcgc ggcu                                    34

<210>  2
<211>  117
<212>  PRT
<213>  Artificial Sequence

<220>
<223>  For VHH antibody

<220>
<221>  MISC_FEATURE
<222>  (1)..(117)

<400>  2

Glu Val Gln Leu Val Glu Ser Gly Gly Gly Leu Val Gln Ala Gly Gly
1               5                   10                  15

Ser Leu Arg Leu Ser Cys Ala Ala Ser Gly Ile Thr Phe Ser Ile Asn
            20                  25                  30

Thr Met Gly Trp Tyr Arg Gln Ala Pro Gly Lys Gln Arg Glu Leu Val
        35                  40                  45

Ala Leu Ile Ser Ser Ile Gly Asp Thr Tyr Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Lys
                85                  90                  95

Arg Phe Arg Thr Ala Gln Gly Thr Asp Tyr Trp Gly Gln Gly Thr Gln
            100                 105                 110

Val Thr Val Ser Ser
        115

<210>  3
<211>  74
<212>  RNA
<213>  Artificial Sequence

<220>
<223>  For RNA aptamer

<220>
<221>  misc_feature
<222>  (1)..(74)

<400>  3
gggagaauuc cgaccagaag uucgauacgc cgugggguga cguuggcuac ccuuuccucu      60

cuccuccuuc uucu                                                        74

<210>  4
<211>  90
<212>  RNA
<213>  Artificial Sequence

<220>
<223>  For RNA aptamer

<220>
<221>  misc_feature
<222>  (1)..(90)

<400>  4
ggacggcacc acggucggau ccgugaguug ugacaauuua gcggguggua uuagagccua      60

cugccacagc aauaggaucg auacagaucu                                      90

<210>  5
<211>  45
<212>  DNA
<213>  Artificial Sequence

<220>
<223>  For DNA aptamer

<400>  5
acgggccaca tcaactcatt gatagacaat gcgtccactg cccgt                      45